(19) Europäisches Patentamt
European Patent Office
Office européen des brevets

(11) EP 0 738 724 B1

(12) EUROPÄISCHE PATENTSCHRIFT

(45) Veröffentlichungstag und Bekanntmachung des Hinweises auf die Patenterteilung:
**04.11.1998 Patentblatt 1998/45**

(51) Int. Cl.$^6$: **C07D 401/06**, C07D 407/06, C07D 411/06

(21) Anmeldenummer: **96112342.9**

(22) Anmeldetag: **01.07.1994**

# (54) Verfahren zur Herstellung eines N-tert.Butylamids

Process for the preparation of an N-terbutylamide derivative

Procédé de préparation d'un dérivé N-terbutylamide

(84) Benannte Vertragsstaaten:
**AT BE CH DE DK ES FR GB IT LI NL**

(30) Priorität: **15.07.1993 CH 2132/93**
**04.08.1993 CH 2330/93**

(43) Veröffentlichungstag der Anmeldung:
**23.10.1996 Patentblatt 1996/43**

(62) Dokumentnummer(n) der früheren Anmeldung(en) nach Art. 76 EPÜ:
**94110247.7 / 0 634 410**

(73) Patentinhaber:
**F. HOFFMANN-LA ROCHE AG**
**4070 Basel (CH)**

(72) Erfinder: **Hilpert, Hans**
**4153 Reinach (CH)**

(74) Vertreter:
**Witte, Hubert, Dr. et al**
**F.Hoffmann-La Roche AG**
**Patent Department (PLP),**
**124 Grenzacherstrasse**
**4070 Basel (CH)**

(56) Entgegenhaltungen:
**EP-A- 0 432 694**

- **JOURNAL OF THE AMERICAN CHEMICAL SOCIETY, Bd. 110, 1988, DC US, Seiten 7538-7539, XP002011973 Y. GAO ET AL.:**
- **TETRAHEDRON LETTERS, Bd. 30, Nr. 20, 1989, OXFORD GB, Seiten 2623-2626, XP002011974 B.B. LOHRAY ET AL.:**
- **CHEMICAL ABSTRACTS, vol. 114, no. 21, 27.Mai 1991 Columbus, Ohio, US; abstract no. 206159q, XP002011976 & JOURNAL OF THE CHEMICAL SOCIETY, CHEMICAL COMMUNICATIONS, Bd. 2, 1991, LETCHWORTH GB, Seiten 95-97, B.B. LOHRAY ET AL.:**
- **PATENT ABSTRACTS OF JAPAN vol. 011, no. 069 (C-407), 3.März 1987 & JP 61 227578 A (NEOSU K.K.)**
- **JOURNAL OF ORGANIC CHEMISTRY, Bd. 59, 1994, EASTON US, Seiten 3656-3664, XP002011975 K.E.B. PARKES ET AL.:**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 738 724 B1

Printed by Xerox (UK) Business Services
2.16.3/3.4

## Beschreibung

Die vorliegende Erfindung betrifft ein neues Verfahren zur Herstellung von N-tert. Butyl-decahydro-2-[2(R)-hydroxy-4-phenyl-3(S)-phthalimidobutyl]-(4aS,8aS)-isochinolin-3(S)-carboxamid der Formel

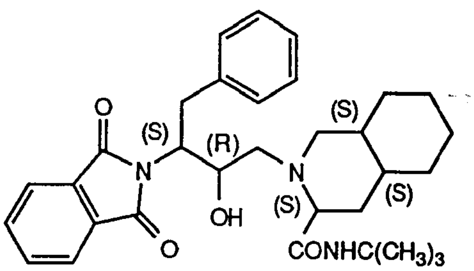

I

sowie neue Zwischenprodukte in dem genannten Verfahren.

Die Verbindung der obigen Formel I ist beispielsweise in Beispiel 1 der Europäischen Patentpublikation 0.432.694 spezifisch beschrieben und ist ein wertvolles Zwischenprodukt zur Herstellung von pharmakologisch aktiven Verbindungen. So kann die Verbindung der Formel I wie in den Beispielen 4 und 5 der genannten Europäischen Patentpublikation beschrieben in pharmakologisch aktive Verbindungen übergeführt werden, welche sich zur Behandlung viraler Infektionen eignen, insbesondere solcher Infektionen, die durch HIV und andere Retroviren verursacht werden.

Das erfindungsgemässe Verfahren ist dadurch gekennzeichnet, dass man ein 1,3,2-Dioxathiolanderivat der allgemeinen Formel

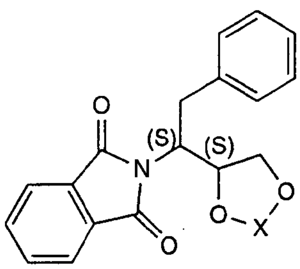

IV

worin X SO oder $SO_2$ bedeutet,
mit der Verbindung der Formel

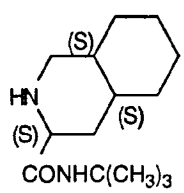

III

umsetzt.

Die Umsetzung einer Verbindung der obigen Formel IV mit der Verbindung der obigen Formel III erfolgt in an sich bekannter Weise in Abhängigkeit von der Bedeutung von X in Formel IV. So kann die Verbindung der Formel IV, worin X SO bedeutet, nach der Methode von B.B. Lohray und J.R. Ahuja [J. Chem. Soc., Chem. Commun. 1991, 95] in Gegenwart einer Base, wie einem Alkylamin, z.B. Triäthylamin, oder einem Alkalicarbonat, z.B. Kalium- oder Natriumcarbonat, und dergleichen in einem höher siedenden, unter den Reaktionsbedingungen inerten organischen Lösungsmittel, wie Isobutylmethylketon und dergleichen bei einer Temperatur von etwa 50°-150°C, vorzugsweise bei etwa 110°C, mit der Verbindung der Formel III umgesetzt werden. Die Verbindung der Formel IV, worin X $SO_2$ bedeutet, kann nach der Methode von Y. Gao und K.B. Sharpless [JACS 110, 7538 (1988)] in einem unter den Reaktionsbedingungen inerten organischen Lösungsmittel, wie einem Aether, z.B. Diäthyläther, Tetrahydrofuran oder Dioxan, und dergleichen bei einer Temperatur zwischen etwa 0° und 80°, vorzugsweise bei Raumtemperatur, mit der Verbindung der Formel III

umgesetzt und anschliessend in Gegenwart einer starken wässrigen Säure, wie einer Mineralsäure, z.B. Schwefelsäure und dergleichen, hydrolysiert werden.

Die in den obigen Verfahren als Ausgangsstoffe eingesetzten Verbindungen der Formel IV sind neu und ebenfalls Gegenstand der vorliegenden Erfindung. Sie können wie im nachstehenden Reaktionsschema formelmässig angegeben hergestellt werden. In dem Reaktionsschema bedeutet R nieder Alkyl oder Phenyl, das durch ein oder zwei Halogenatome substituiert sein kann.

Die als Ausgangsstoff eingesetzte Verbindung der Formel III ist bekannt und entspricht derjenigen der Formel VII in der Europäischen Patentpublikation 0.432.695.

Der oben verwendete Ausdruck "nieder-Alkyl" betrifft geradkettige und verzweigte gesättigte Kohlenwasserstoffreste mit 1-6, vorzugsweise 1-4, Kohlenstoffatomen, wie Methyl, Aethyl, n-Propyl, Isopropyl, n-Butyl, Isobutyl, sec.Butyl, tert.Butyl, Pentyl, Hexyl und dergleichen. Der Ausdruck "Halogen" betrifft Fluor, Chlor, Brom und Jod.

Reaktionsschema

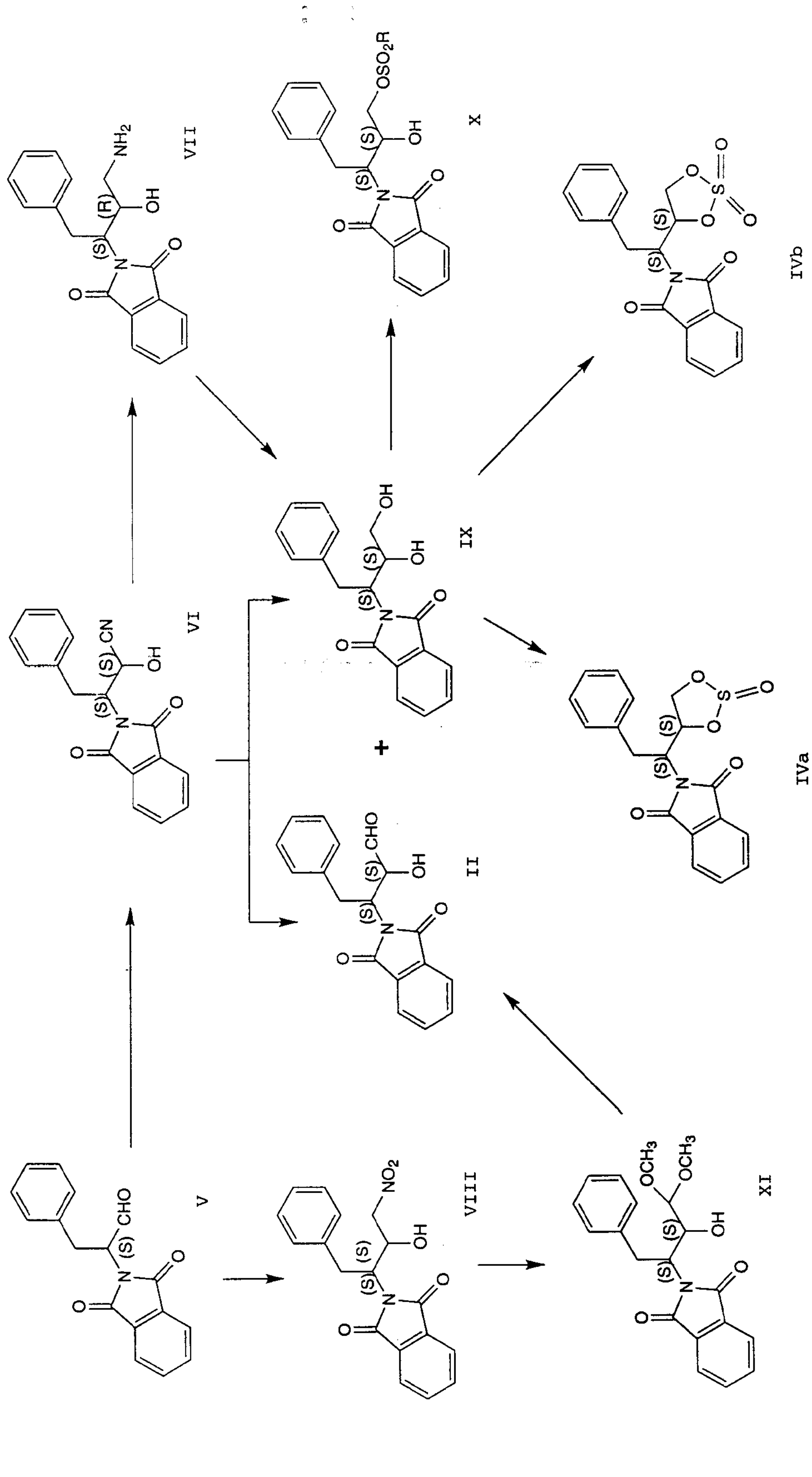

VII X IVb VI IX IVa II V VIII XI

Das 1,3,2-Dioxathiolanderivat der Formel IV kann vom 3-Phenyl-2(S)-phthalimidopropan-1-al der Formel V ausgehend gemäss der Sequenz V→VI→VII→IX→IV erhalten werden. Dabei wird der Aldehyd der Formel V im ersten Schritt in an sich bekannter Weise in das (2S,3S)-3-(1,3-Dioxo-2,3-dihydro-1H-isoindol-2-yl)-2-hydroxy-4-phenylbutyronitril

der Formel VI übergeführt. Dazu wird eine Lösung des Aldehyds der Formel V, z.B. in Toluol, mit wässerigem Natriumpyrosulfit versetzt, und das entstandene Additionsprodukt von Pyrosulfit und dem Aldehyd, gegebenenfalls in einem Lösungsmittel, wie Wasser oder gegebenenfalls wässrigem Dichlormethan oder Toluol, mit Natriumcyanid behandelt. In einer Variante wird ein Gemisch des Aldehyds der Formel V und Zink-(II)-bromid in einem Lösungsmittel, wie Methylenchlorid, bei -70° bis 0°C, z.B. -15°C, mit Trimethylsilylcyanid umgesetzt, und der entstandene Silyläther des Cyanhydrins durch Zugabe einer Lösung von Zitronensäure in Aethanol gespalten.

Im nächsten Schritt wird das erhaltene Cyanhydrin der Formel VI in ebenfalls bekannter Weise in das (1S,2R)-2-(3-Amino-1-benzyl-2-hydroxypropyl)-2,3-dihydro-1H-isoindol-1,3-dion der Formel VII übergeführt, und zwar durch Hydrierung in Gegenwart eines Katalysators, wie Platinoxid, Palladium oder Raney-Nickel, vorzugsweise Platinoxid, und Umsetzen mit einer Säure, wie einer Mineralsäure, Sulfonsäure oder Alkancarbonsäure, vorzugsweise Essigsäure, in einem Lösungsmittel, wie einem Alkohol oder einer Alkancarbonsäure, vorzugsweise Essigsäure. Die Hydrierung erfolgt bei einem Druck von etwa 100-5000 kPa, vorzugsweise etwa 100 kPa, und bei einer Temperatur zwischen etwa -15° und 80°C, vorzugsweise bei Raumtemperatur.

Anschliessend wird der Aminoalkohol der Formel VII ebenfalls in an sich bekannter Weise in das Diol der Formel IX, das (1S,2S)-2-(1-Benzyl-2,3-dihydroxypropyl)-2,3-dihydro-1H-isoindol-1,3-dion, übergeführt. Diese Ueberführung via Diazoniumsalz erfolgt ebenfalls nach an sich bekannten Methoden mit Natriumnitrit in Gegenwart einer wässerigen Mineralsäure oder Carbonsäure, vorzugsweise in einem 1:1-Gemisch von Essigsäure und Wasser, bei einer Temperatur zwischen etwa 0° und 40°C, vorzugsweise bei etwa 0°C. Der Aminoalkohol der Formel VII braucht vor der Ueberführung in das Diol der Formel IX nicht isoliert zu werden; vielmehr erfolgt die Sequenz VI→VII→IX zweckmässigerweise in einem Topf.

Gemäss einer Alternative kann die Hydrierung des Cyanhydrins der Formel VI in Gegenwart von mindestens je 1 Moläquivalent Säure und Wasser durchgeführt werden. In diesem Fall wird direkt ein Gemisch aus dem Aldehyd der Formel II und dem Diol der Formel IX erhalten, wobei das Verhältnis von den verwendeten Reaktionsbedingungen abhängt. Auch diese Reaktionsführung erfolgt nach an sich bekannten Methoden in Gegenwart von einem Katalysator, wie Palladium, Platinoxid oder Raney-Nickel, vorzugsweise Palladium auf Kohle, und je 1 Moläquivalent einer anorganischen oder organischen Säure, wie Salzsäure oder Essigsäure und dergleichen, oder eines sauren Ionenaustauschers, wie Dowex 50Wx4,$H^+$ und dergleichen, und Wasser, in einem unter den Reaktionsbedingungen inerten organischen Lösungsmittel, wie einem Alkohol, z.B. Isopropanol, und dergleichen bei einer Temperatur zwischen etwa -15° und 100°C, zweckmässigerweise bei Raumtemperatur, und einem Druck von etwa 100-15000 kPa, vorzugsweise 100-1000 kPa.

Das Diol der Formel IX wird dann in einem weiteren Schritt in ein 1,3,2-Dioxathiolanderivat der Formel IV übergeführt. Wird die Verbindung der Formel IVa erwünscht, so wird das Diol der Formel IX in an sich bekannter Weise in Gegenwart einer organischen Base, vorzugsweise einem Amin, wie einem Alkylamin, z.B. Triäthylamin oder Hünigbase, in einem unter den Reaktionsbedingungen inerten organischen Lösungsmittel, wie einem Aether, z.B. Diäthyläther, Tetrahydrofuran oder Dioxan, und dergleichen bei einer Temperatur zwischen etwa -15° und 80°C, vorzugsweise bei etwa 0°C, mit Thionylchlorid umgesetzt. Dabei wird ein 1:1-Gemisch der beiden Isomeren 2-[(S)-1-[(2R,4S)- und [(2S,4S)-2-Oxo-1,3,2-dioxathiolan-4-yl]-2-phenyläthyl]-2,3-dihydro-1H-isoindol-1,3-dion erhalten. Wird hingegen die Verbindung der Formel IVb erwünscht, so erfolgt die Umsetzung ebenfalls in an sich bekannter Weise mit Thionylchlorid ohne Zusatz einer organischen Base bei einer Temperatur zwischen etwa Raumtemperatur und 80°C, vorzugsweise bei etwa 80°C, in einem unter den Reaktionsbedingungen inerten organischen Lösungsmittel, wie einem chlorierten Kohlenwasserstoff, z.B. Tetrachlorkohlenstoff, und dergleichen. Das Rohprodukt wird in einem zweiten Reaktionsschritt in einem Lösungsmittelgemisch, wie z.B. Tetrachlorkohlenstoff/Acetonitril/Wasser, und dergleichen mit einem Oxidationsmittel umgesetzt. Auch dieser Reaktionsschritt erfolgt nach an sich bekannten Methoden. Ein für den Zweck der vorliegenden Erfindung geeignetes Oxidationsmittel ist z.B. eine stöchiometrische Menge Natriumperjodat in Gegenwart einer katalytischen Menge von Rutheniumchlorid bei einer Temperatur zwischen etwa 0° und 50°C, vorzugsweise bei Raumtemperatur.

Ein Alternativverfahren zur Herstellung der Verbindung der Formel I,besteht darin, daß das Diol der Formel IX in an sich bekannter Weise mit einem Sulfonylchlorid der allgemeinen Formel

$$RSO_2Cl$$

worin R die oben angegebene Bedeutung besitzt,

in Gegenwart einer organischen Base, wie Pyridin, Piperidin, einem Alkylamin, z.B. Triäthylamin, und dergleichen in einem unter den Reaktionsbedingungen inerten organischen Lösungsmittel, wie einem aromatischen Kohlenwasserstoff, z.B. Toluol oder Xylol, einem Alkanearbonsäureester, z.B. Aethylacetat, und dergleichen in eine Verbindung der Formel X übergeführt wird, welche dann anschliessend nach ebenfalls an sich bekannten Methoden in Gegenwart einer starken Base, wie einem Alkalimetallhydrid, z.B. Natriumhydrid, oder einem Alkalimetallalkoxid, z.B. Kalium-tert.butylat, und dergleichen in einem unter den Reaktionsbedingungen inerten organischen Lösungsmittel, wie Dimethylformamid oder einem Aether, z.B. Diäthyläther, Tetrahydrofuran oder Dioxan, und dergleichen bei einer Temperatur zwischen etwa -15° bis 50°C, vorzugsweise bei etwa -15°C, in das Epoxyd 2(S)-[2-Phenyl-1(S)-phthalimidoäthyl]oxiran der Formel

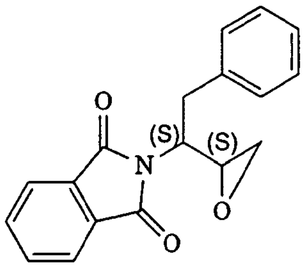

XII

übergefürht wird.

Das Epoxyd der Formel XII sowie dessen Ueberführung in die Verbindung der Formel I ist spezifisch bekannt und im Beispiel 1 der Europäischen Patentpublikation 0.432.694 beschrieben.

Das 3-Phenyl-2(S)-phthalimidopropan-1-al der Formel V erhält man durch Erhitzen von L-Phenylalanin mit Phthalsäureanhydrid in Toluol, Umsetzung des entstandenen N-geschützten L-Phenylalanins mit Oxalylchlorid in Toluol und katalytischen Mengen Dimethylformamid und katalytischer (Pd/C) Hydrierung des entstandenen, dem erwünschten Aldehyd der Formel V entsprechenden Säurechlorids in Gegenwart von 1,2-Butylenoxid in Toluol.

Die folgenden Beispiele sollen die vorliegende Erfindung erläutern, sie jedoch in keiner Weise einschränken. Alle Temperaturen sind in Celsiusgraden angegeben.

Beispiel 1

A1) Eine Suspension von 5 g 3-Phenyl-2(S)-phthalimidopropan-1-al und 4,43 g Zinkbromid in 50 ml Methylenchlorid wird unter Rühren bei -15° mit einer Lösung von 1,95 g Trimethylsilylcyanid in 5 ml Methylenchlorid versetzt und 5 Stunden bei -15° gerührt. Der gebildete Silyläther wird durch Zugabe einer Lösung von 5 g Zitronensäure in 50 ml Aethanol bei -10° gespalten. Das Gemisch wird eingeengt, und der Rückstand mit Wasser versetzt und mit Methylenchlorid extrahiert. Die organischen Extrakte werden getrocknet, filtriert, und das Filtrat eingeengt. Der Rückstand enthält 5,45 g (99%) rohes 3-(1,3-Dioxo-2,3-dihydro-1H-isoindol-2-yl)-2-hydroxy-4-phenylbutyronitril als 74:26-Gemisch der (2S,3S)- und (2R,3S)-Isomeren, IR (KBr): 3437m (OH), 2250w (C≡N), 1775m und 1713s (C=O von Imid).

A2) Der das 3-Phenyl-2(S)-phthalimidopropan-1-al enthaltenden Lösung wird bei Raumtemperatur unter Rühren eine Lösung von 95,05 g Natriumpyrosulfit in 1 l Wasser zugesetzt. Nach 4,5 Stunden Rühren wird die das Additionsprodukt von Bisulfit und des obigen Aldehyds enthaltende wässrige Schicht mit Toluol gewaschen. Die Toluolschichten werden mit Wasser extrahiert. Den Wasserschichten werden 1200 ml Methylenchlorid zugesetzt, und das Gemisch wird unter Rühren bei Raumtemperatur mit einer Lösung von 41,66 g Natriumcyanid in 330 ml Wasser versetzt. Nach 1,2 Stunden Rühren wird Wasser zugesetzt. Die abgetrennte wässrige Schicht wird mit Methylenchlorid extrahiert. Die organischen Schichten werden getrocknet und filtriert, und der Rückstand wird mit Methylenchlorid gewaschen. Die Filtrate werden eingedampft, und der Rückstand in 200 ml Methylenchlorid gelöst. Der Lösung werden unter Rühren bei 30° 600 ml Hexan und dann bei 0° nochmals 600 ml Hexan zugesetzt. Die Suspension wird filtriert, und der Rückstand mit Hexan gewaschen und dann getrocknet. Man erhält 114,02 g (74%) eines 74,7:23,5:1,4:0,4-Gemisches der Isomeren (2S,3S):(2R,3S):(2R,3R):(2S,3R) von 3-(1,3-Dioxo-2,3-dihydro-1H-isoindol-2-yl)-2-hydroxy-4-phenylbutyronitril, Smp. 127,2-130,5°, $[\alpha]_D^{20}$ : -146,6° (1% in Methylenchlorid).

A3) Der das 3-Phenyl-2(S)-phthalimidopropan-1-al enthaltenden Lösung wird bei Raumtemperatur unter Rühren eine Lösung von 47,5 g Natriumpyrosulfit in 500 ml Wasser zugesetzt. Nach 7,5 Stunden Rühren wird die das Additionsprodukt von Pyrosulfit und des obigen Aldehyds enthaltende wässrige Schicht mit Toluol gewaschen. Die Toluolschichten werden mit Wasser extrahiert. Den Wasserschichten wird unter Rühren bei Raumtemperatur eine Lösung von 24,2 g Natriumcyanid in 200 ml Wasser zugesetzt. Die Suspension wird nach 1 Stunde Rühren filtriert, und der Rückstand mit Wasser neutral gewaschen. Nach Trocknen erhält man 112,03 g (73%) eines 67,2;32,8-Gemisches der Isomeren (2S,3S):(2R,3S) von 3-(1,3-Dioxo-2,3-dihydro-1H-isoindol-2-yl)-2-hydroxy-4-phenylbutyronitril, Smp. 131-133°, $[\alpha]_D^{20}$ : -150,2° (1% in Methylenchlorid).

B) Eine Suspension von 20,0 g eines 75:25-Gemisches der (2S,3S)- und (2R,3S)-Isomeren von 3-(1,3-Dioxo-2,3-dihydro-1H-isoindol-2-yl)-2-hydroxy-4-phenylbutyronitril und 2 g Platinoxid in 200 ml Essigsäure werden 2,5 Stunden bei Raumtemperatur und Atmosphärendruck hydriert, wobei 4000 ml Wasserstoff aufgenommen werden. Die Suspension wird mit 3 g Aktivkohle versetzt, 1 Stunde gerührt und filtriert. Das Filtrat, welches (1S,2R)-2-(3-Amino-1-benzyl-2-hydroxypropyl)-2,3-dihydro-1H-isoindol-1,3-dion-acetat enthält, wird mit 200 ml Wasser verdünnt, auf 0° abgekühlt, mit einer Lösung von 4,50 g Natriumnitrit in 6 ml Wasser versetzt, 3 Stunden bei 0° gerührt, erneut mit einer Lösung von 4,50 g Natriumnitrit in 6 ml Wasser versetzt und schliesslich nochmals 2 Stunden bei 0° gerührt. Das Reaktionsgemisch wird danach mit Methylenchlorid extrahiert. Die Extrakte werden mit Wasser gewaschen, getrocknet und filtriert, und das Filtrat eingeengt. Der Rückstand wird mit Methylenchlorid/Isopropanol (100:1) an Kieselgel gereinigt. Nach dem Eindampfen und Trocknen erhält man 6,25 g (31%) eines 83:17-Gemisches der (1S,2S)- und (1S,2R)-Isomeren von 2-(1-Benzyl-2,3-dihydroxypropyl)-2,3-dihydro-1H-isoindol-1,3-dion, IR: 3460 (OH), 1771m und 1701s (C=O von Imid).

C) Eine Lösung von 0,51 g eines 83:17-Gemisches der (1S,2S)- und (1S,2R)-Isomeren von 2-(1-Benzyl-2,3-dihydroxypropyl)-2,3-dihydro-1H-isoindol-1,3-dion und 0,28 ml Triäthylamin in 5 ml Tetrahydrofuran wird auf 0° gekühlt, mit 0,15 ml Thionylchlorid versetzt und 1 Stunde bei 0° gerührt. Das Reaktionsgemisch wird mit 4 ml halbgesättigter Kochsalzlösung und 1 ml 1N Schwefelsäure versetzt und mit Essigester extrahiert. Die Extrakte werden getrocknet und filtriert, und das Filtrat zu 0,60 g (100%) von 83 Teilen eines 1:1 Gemisches der (2R,4S)- und (2S,4S)-Isomeren von 2-[(S)-1-[2-Oxo-1,3,2-dioxathiolan-4-yl]-2-phenyläthyl]-2,3-dihydro-1H-isoindol-1,3-dion sowie 17 Teilen eines 1:1 Gemisches der (2R,4R)- und (2S,4R)-Isomeren von 2-[(S)-1-[2-Oxo-1,3,2-dioxathiolan-4-yl]-2-phenyläthyl]-2,3-dihydro-1H-isoindol-1,3-dion eingedampft, IR (KBr): 1777m und 1713s (C=O von Imid), 1384s und 1212s ($SO_3$).

D) Eine Suspension von 0,35 g des obigen 83:17-Gemisches der beiden 1:1-Gemische der (2R,4S)- und (2S,4S)- bzw. (2R,4R)- und (2S,4R)-Isomeren von 2-[(S)-1-[2-Oxo-1,3,2-dioxathiolan-4-yl]-2-phenyläthyl]-2,3-dihydro-1H-isoindol-1,3-dion, 0,24 g N-tert.Butyl-decahydro-(4aS,8aS)-isochinolin-3(S)-carboxamid und 0,21 g Natriumcarbonat in 3,5 ml Isobutylmethylketon wird 24 Stunden zum Rückfluss erhitzt, danach abgekühlt und filtriert. Das Filtrat wird mit Hexan/Aethylacetat (4:1) an Kieselgel gereinigt. Nach dem Eindampfen und Trocknen erhält man 0,34 g (65%) reines N-tert.Butyl-decahydro-2-[2(R)-hydroxy-4-phenyl-3(S)-phthalimidobutyl]-(4aS,8aS)-isochinolin-3(S)-carboxamid, IR (KBr): 3388m (OH), 1771m und 1708s (C=O von Imid).

Beispiel 2

A) Ein Gemisch von 3,1 g eines 83:17-Gemisches der (1S,2S)- und (1S,2R)-Isomeren von 2-(1-Benzyl-2,3-dihydroxypropyl)-2,3-dihydro-1H-isoindol-1,3-dion und 10 ml Tetrachlorkohlenstoff wird mit 0,9 ml Thionylchlorid versetzt und 0,5 Stunden zum Rückfluss erhitzt. Das Gemisch wird bei 0° mit 10 ml Acetonitril verdünnt und nacheinander mit 2 mg Rutheniumtrichloridtrihydrat, 3,2 g Natriumperjodat und 15 ml Wasser versetzt und 1 Stunde bei Raumtemperatur gerührt. Das Gemisch wird mit Aether extrahiert, und die Extrakte werden mit verdünnter Natriumbicarbonatlösung und Wasser gewaschen. Trocknen, Filtrieren und Einengen des Filtrates liefert ein 83:17-Gemisch von 2-[(S)-1-[(4S)- und (4R)-2-Dioxo-1,3,2-dioxathiolan-4-yl]-2-phenyläthyl]-2,3-dihydro-1H-isoindol-1,3-dion.

B) Eine Lösung von 0,37 g eines 83:17-Gemisches von 2-[(S)-1-[(4S)- und (4R)-2-Dioxo-1,3,2-dioxathiolan-4-yl]-2-phenyläthyl]-2,3-dihydro-1H-isoindol-1,3-dion und 0,24 g N-tert.Butyl-decahydro-(4aS,8aS)-isochinolin-3(S)-carboxamid in 2 ml Tetrahydrofuran wird bis zum vollständigen Umsatz gerührt, anschliessend mit 5 ml 20%iger Schwefelsäure und 5 ml Aether versetzt, 10 Stunden gerührt und mit Aether extrahiert. Die Extrakte werden mit Wasser gewaschen, getrocknet und filtriert, und das Filtrat zu N-tert.Butyl-decahydro-2-[2(R)-hydroxy-4-phenyl-3(S)-phthalimidobutyl]-(4aS,8aS)-isochinolin-3(S)-carboxamid eingedampft, IR (KBr): 3388m (OH), 1771m und 1708s (C=O von Imid).

Beispiel 3

A) Eine Lösung von 0,51 g eines 83:17-Gemisches der (1S,2S)- und (1S,2R)-Isomeren von 2-(1-Benzyl-2,3-dihydroxypropyl)-2,3-dihydro-1H-isoindol-1,3-dion, 0,4 ml Pyridin und 1 ml Aethylacetat wird mit 0,15 ml Methansulfochlorid versetzt und 4 Stunden bei Raumtemperatur gerührt. Das Gemisch wird mit Aethylacetat verdünnt und mit 2% Salzsäure gewaschen, und die Extrakte getrocknet und filtriert. Das Filtrat wird eingedampft, und der Rückstand mit Methylenchlorid/Isopropanol (100:1) an Kieselgel gereinigt. Nach dem Eindampfen und Trocknen erhält man 0,28 g (43%) reinen Methansulfonsäure-(2S,3S)-3-(1,3-Dioxo-2,3-dihydro-1H-isoindol-2-yl)-2-hydroxy-4-phe-

nylbutylester, NMR ($CDCl_3$): 7,7 (m,4H); 7,1 (m,5H); 4,6 (m,2H); 4,3 (m,2H); 3,55 (d,J=7,1H); 3,3 (m,2H); 3,1 (s,3H).

B) Eine Lösung von 0,26 g Methansulfonsäure-(2S,3S)-3-(1,3-Dioxo-2,3-dihydro-1H-isoindol-2-yl)-2-hydroxy-4-phenylbutylester in 1,6 ml Tetrahydrofuran wird bei -15° mit einer Lösung von 0,09 g Kalium-tert.butylat in 0,5 ml Tetrahydrofuran versetzt und 0,5 Stunden gerührt. Das Gemisch wird mit 2,2 ml halbgesättigter Kochsalzlösung versetzt, und der pH-Wert mit 0,1 ml 1N Schwefelsäure auf 6 gestellt. Danach wird mit Aethylacetat extrahiert. Die Extrakte werden getrocknet und filtriert, und das Filtrat zu 0,12 g (60%) 2(S)-[2-Phenyl-1(S)-phthalimidoäthyl]oxiran eingedampft, IR (KBr): 1773m und 1709s (C=O von Imid).

## Patentansprüche

1. Verfahren zur Herstellung von N-tert.Butyl-decahydro-2-[2(R)-hydroxy-4-phenyl-3(S)-phthalimidobutyl]-(4aS,8aS)-isochinolin-3(S)-carboxamid der Formel

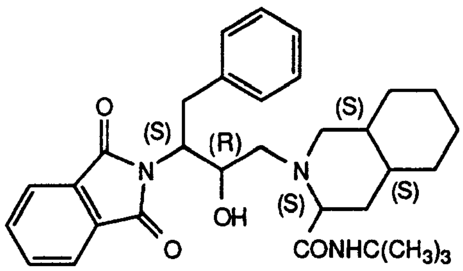

I

dadurch gekennzeichnet, dass man ein 1,3,2-Dioxathiolanderivat der allgemeinen Formel

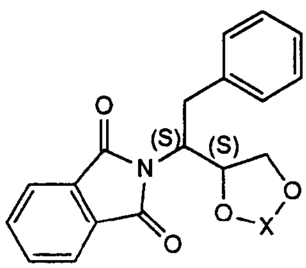

IV

worin X SO oder $SO_2$ bedeutet,
mit der Verbindung der Formel

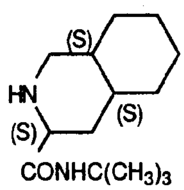

III

umsetzt.

2. 2-[(S)-1-[(2R,4S)- und [(2S,4S)-2-Oxo-1,3,2-dioxathiolan-4-yl]-2-phenyläthyl]-2,3-dihydro-1H-isoindol-1,3-dion der Formel

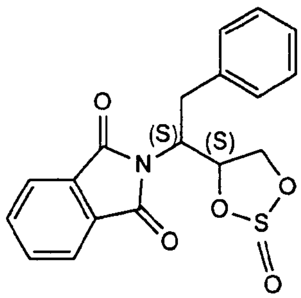

IVa

3. 2-[(S)-1-[(4S)-2-Dioxo-1,3,2-dioxathiolan-4-yl]-2-phenyläthyl]-2,3-dihydro-1H-isoindol-1,3-dion

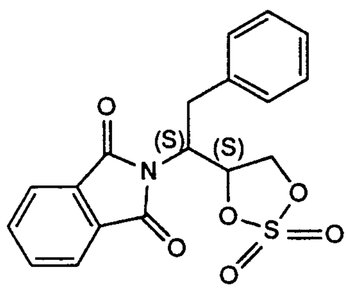

IVb

## Claims

1. A process for the manufacture of N-tert.butyl-decahydro-2-[2(R)-hydroxy-4-phenyl-3(S)-phthalimidobutyl]-(4aS,8aS)-isoquinoline-3(S)-carboxamide of the formula

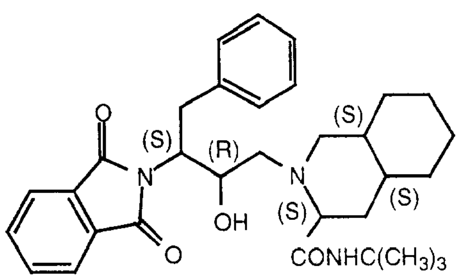

I

characterized by reacting a 1,3,2-dioxathiolane derivative of the general formula

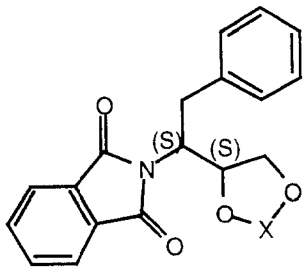

IV

wherein X signifies SO or $SO_2$,
with the compound of the formula

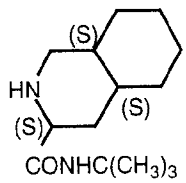

III

**2.** 2-[(S)-1-[(2R,4S)- and [(2S,4S)-2-oxo-1,3,2-dioxathiolan-4-yl]-2-phenylethyl]-2,3-dihydro-1H-isoindole-1,3-dione of the formula

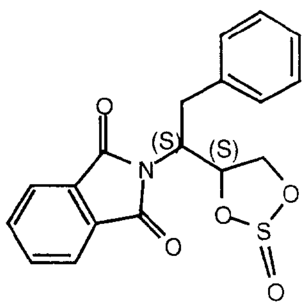

IVa

**3.** 2-[(S)-1-[(4S)-2-Dioxo-1,3,2-dioxathiolan-4-yl]-2-phenylethyl]-2,3-dihydro-1H-isoindole-1,3-dione

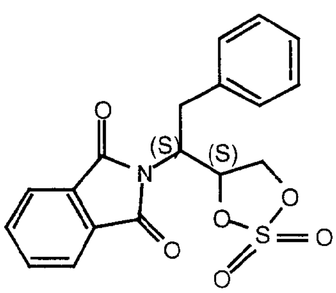

IVb

## Revendications

1. Procédé de préparation du N-tert.-butyl-décahydro2-[2(R)-hydroxy-4-phényl-3(S)-phtalimidobutyl]-(4aS,8aS)-isoquinoléin-3(S)-carboxamide de formule

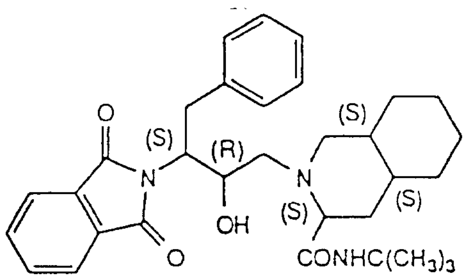

I

caractérisé en ce que l'on met à réagir un dérivé 1,3,2-dioxathiolane de formule générale

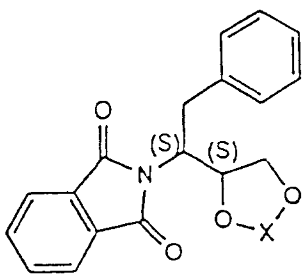

IV

dans laquelle X représente SO ou $SO_2$,
avec le composé de formule

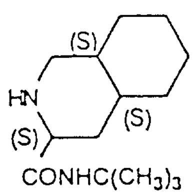

III

2. 2-[(S)-1-[(2R,4S)- et [(2S,4S)-2-oxo-1,3,2-dioxathiolan-4-yl]-2-phényléthyl]-2,3-dihydro-1H-isoindol-1,3-dione de formule

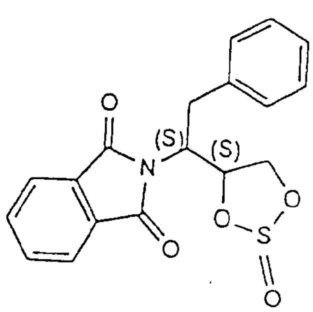

IVa

3. 2-[(S)-1-[(4S)-2-dioxo-1,3,2-dioxathiolan-4-yl]-2-phényléthyl]-2,3-dihydro-1H-isoindol-1,3-dione

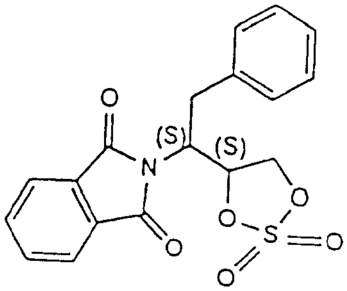

IVb